# EUROPEAN PATENT APPLICATION

(11) **EP 4 138 421 A1**
(43) Date of publication of application: **22.02.2023**
(21) Application number: 22191039.1
(22) Date of filing: 18.08.2022
(51) Int. Cl.: H04W 4/02, H04W 4/21, G06F 3/0482, G16H 40/20, H04W 4/80

(54) **MANAGING CAREGIVER COMMUNICATIONS**

(30) Priority: 19.08.2021 US 202163234777 P
(71) Applicant: Hill-Rom Services, Inc., Batesville, IN 47006-9167 (US)
(72) Inventor: WIGGERMANN, Neal, Batesville, 47006-9167 (US)
(74) Representative: Bauer, Dustin

(57) **Abstract**

A system for managing caregiver communications in a healthcare facility is described. The system detects an interaction between a first caregiver and a second caregiver, and determines whether the second caregiver is a contact of the first caregiver. The system determines whether the first caregiver is permitted to receive the contact of the second caregiver based on at least the interaction in the healthcare facility. The system sends an invitation to a mobile device of the first caregiver, the invitation allowing the first caregiver to accept or decline adding the contact of the second caregiver when the first caregiver is permitted to receive the contact of the second caregiver.

## Description

Caregivers, such as doctors, nurses, physician assistants, and the like are typically assigned to multiple patients during a shift in a healthcare facility such as a hospital. Caregivers often cross paths with one another and with other staff members during their shifts.

Caregivers typically know the names and have the contact information of other caregivers and staff members with whom they regularly work with during their shifts. However, caregivers may not know the names or have the contact information of other caregivers who visit on a temporary basis or who infrequently visit the healthcare facility.

In general terms, the present disclosure relates to improving communications between caregivers in a healthcare facility. In one possible configuration, a system detects caregiver interactions inside a healthcare facility, and sends invitations for caregivers to store contact information of other caregivers based on the interactions. Various aspects are described in this disclosure, which include, but are not limited to, the following aspects.

One aspect relates to a system for managing caregiver communications in a healthcare facility, the system comprising: at least one processing device; and a memory device storing instructions which, when executed by the at least one processing device, cause the system to: detect an interaction between a first caregiver and a second caregiver; determine whether the second caregiver is a contact of the first caregiver; determine whether the first caregiver is permitted to receive the contact of the second caregiver based on at least the interaction in the healthcare facility; and send an invitation to a mobile device of the first caregiver, the invitation allowing the first caregiver to accept or decline adding the contact of the second caregiver when the first caregiver is permitted to receive the contact of the second caregiver.

Optionally, determine whether the first caregiver is permitted to receive the contact information of the second caregiver is based on at least one of a unit, a team, a department, a patient, a patient room, and a shift assigned to the first and second caregivers.

Optionally, determine whether the first caregiver is permitted to receive the contact information of the second caregiver is based on the second caregiver's preferences.

Optionally, the memory device stores further instructions which, when executed by the at least one processing device, cause the system to: display an interaction timeline on the mobile device of the first caregiver, the interaction timeline including interactions with other caregivers in the healthcare facility.

Optionally, the interaction timeline is filtered to include only interactions with the other caregivers who are not contacts of the first caregiver. Alternatively, the interactions with the other caregivers who are contacts of the first caregiver are displayed on the interaction timeline differently from the interactions with the other caregivers who are not contacts of the first caregiver.

Optionally, interactions displayed on the interaction timeline may be selectable to receive the contact information of the caregiver or person associated with the respective interaction.

Optionally, the contact information may be automatically obtained from a, or the, contact information database and added to the stored contacts upon selection of an icon. Alternatively, an invitation may be sent to the caregiver or person associated with the selected icon, and the caregiver or person can accept or decline the invitation to share their contact information with the caregiver associated with the mobile device.

Optionally, the interaction is detected by receiving signals from one or more fixed reference points indicating the first and second caregivers are located together in a predefined area, or have visited the predefined area within a predetermined time window. Further optionally, the one or more fixed reference points obtain the signals from badges worn by the first and second caregivers.

Optionally, the or each fixed reference point comprises a sensor. Optionally, the or each fixed reference point comprises a camera.

Optionally, the predefined area may be defined based on a predetermined proximity to a plurality of sensors. Alternatively, the predefined area may be defined based on a fixed reference point that is equidistant to a plurality of sensors.

Optionally, the interaction is detected by receiving a video stream and using facial recognition to identify the first and second caregivers as being located together in a predefined area, or having visited the predefined area within a predetermined time window.

Optionally, the interaction is detected by receiving data from the mobile device of the first caregiver and a mobile device of the second caregiver.

Optionally, the data includes GPS signals indicating the first and second caregivers are located together in a predefined area, or have visited the predefined area within a predetermined time window and/or wherein the data includes signals obtained from fixed reference points indicating the first and second caregivers are located together in a predefined area, or have visited the predefined area within a predetermined time window.

Optionally, the interaction is detected by receiving data from the mobile device of the first caregiver detecting a mobile device of the second caregiver using at least one of Wi-Fi, ultra-wideband (UWB), Bluetooth, and radio frequency identification (RFID).

Optionally, the interaction is detected by receiving data from badges worn by the first and second caregivers, and the data includes signals obtained from fixed reference points indicating the first and second caregivers are located together in a predefined area, or have visited the predefined area within a predetermined time window.

Optionally, the badges comprise a wireless real-time locating systems (RTLS) tag configured to be detectable by a, or the, sensor. In examples where the, or each, fixed reference point comprises a sensor, the fixed reference point can detect and receive wireless signals from RTLS tags to determine the location of the caregivers.

The RTLS tags may be configured to emit radio frequency (RF) signals, optical (e.g., infrared) signals, or acoustic (e.g., ultrasound) signals. The, or each, sensor may be configured to detect radio frequency (RF) signals, optical (e.g., infrared) signals, or acoustic (e.g., ultrasound) signals from the badges.

Optionally or alternatively, the, or each, fixed reference point may be configured to receive short range data signals from the badges that indicate the presence of the caregivers inside the predefined area. For example, the short range data signals may be ID signals from the badges.

Optionally, the memory device stores further instructions which, when executed by the at least one processing device, cause the system to: relay the data signals (e.g. ID signals, RTLS signals) to a mobile application server. The system may comprise the mobile application server.

The fixed reference points may be configured to transmit the detected wireless signals to the mobile application server for analysis. Optionally, the mobile application server is configured to determine a location of the caregivers in the healthcare facility, and thereby determine an interaction when two or more caregivers are located in the same area, from the wireless signals from the RTLS tags.

Optionally, the system, or the memory device, includes a contact information database. The contact information database may be configured to store contact information of the caregivers such as phone number, email address, office/desk location, and the like. The contact information database may also be configured to store contact information of additional staff members in the healthcare facility such as administrators, staff members, and the like.

Optionally, the invitation may include an interaction, which may include a caregiver identity of the second caregiver, a time stamp of when the interaction took place, and any patients for which both caregivers share responsibility. In some examples, the interaction may also identify a location of where the interaction took place. The invitation may include a profile image of the second caregiver.

Optionally, determine whether the first caregiver is permitted to receive the contact of the second caregiver based on at least the interaction in the healthcare facility may comprise determining whether one or more rules is satisfied. The one or more rules may comprise default rules, facility rules, and caregiver preferences. Default rules may be are general rules that govern communications between the caregivers, and facility rules may be rules set by the healthcare facility and may supplement the default rules such as by adding additional rules, or overriding one or more of the default rules.

Optionally, the default rules or the facility rules may comprise: an invitation to store the contact information of the second caregiver is sent to the mobile device of the first caregiver only when the first caregiver interacts with the second caregiver within the predefined area of the healthcare facility, such as a patient room. Advantageously, this may avoid sending invitations when there is a meaningless interaction, such as when the first caregiver interacts with the second caregiver in a common area.

Optionally, the default rules or the facility rules may comprise: an invitation is sent only after the first caregiver has interacted with the second caregiver beyond a predetermined number of times (e.g., only after the third interaction) in the healthcare facility. Optionally, the predetermined number of interactions must occur within a predetermined time frame (e.g., three interactions with the second caregiver within one week). Advantageously, this may avoid sending invitations when there is a one-off interaction, such as when the first caregiver interacts in the healthcare facility with the second caregiver only once, and never again.

Optionally, the default rules or the facility rules may comprise: blocking a first caregiver from receiving the contact information of a second caregiver based on at least one of assigned department or unit within the healthcare facility, assigned patients, patient rooms, or patient areas within the healthcare facility, or scheduled shifts in the healthcare facility. For example, the one or more rules defined by the default rules or facility rules may allow sharing contact information only between caregivers who are in the same department or unit within the healthcare facility.

Alternatively or optionally, the rules may allow sharing contact information only between caregivers who are assigned to at least one common patient or who are assigned to at least one common patient room or patient area within the healthcare facility or between caregivers who have overlapping shifts in the healthcare facility.

Optionally, the instructions further cause the system to check an electronic medical record (EMR) of a patient to determine whether the first and second caregivers are caring for the same patient, or whether the second caregiver would be likely to interact with the first caregiver based on one or more medical conditions of the patient identified from the patient's EMR. Such rules may help anticipate or predict a need for the first caregiver to contact the second caregiver.

Another aspect relates to a mobile device for managing caregiver communications in a healthcare facility, the mobile device comprising: at least one processing device; and a memory device storing instructions which, when executed by the at least one processing device, cause the mobile device to: detect a signal from a second mobile device associated with a caregiver; determine whether the caregiver is a contact; determine whether the mobile device is permitted to receive the contact of the caregiver based on at least an interaction with the caregiver in the healthcare facility; and provide an invitation to add the contact of the caregiver when the mobile device is permitted to receive the contact of the caregiver.

Another aspect relates to a method for managing caregiver communications in a healthcare facility, the method comprising: detecting an interaction between a first caregiver and a second caregiver; determining whether the second caregiver is a contact of the first caregiver; determining whether sharing the contact of the second caregiver is permitted based on at least one of a unit, a team, a department, a patient, a patient room, and a shift assigned to the first and second caregivers; and sending an invitation to a mobile device of the first caregiver, the invitation allowing the first caregiver to add the contact of the second caregiver.

Another aspect relates to a method for managing caregiver communications in a healthcare facility, the method comprising: detecting an interaction between two or more caregivers in the healthcare facility; determining whether a display of the interaction is permitted based on one or more shared characteristics between the two or more caregivers including assigned department or unit within the healthcare facility, assigned patients, patient rooms, or patient areas within the healthcare facility, or scheduled shifts in the healthcare facility; and displaying the interaction on a mobile device associated with a first caregiver when the first caregiver has the one or more shared characteristics.

The invention will now be further described by way of example with reference to the accompanying drawings, in which:
FIG. 1 illustrates an example of a caregiver communications system for managing communications between caregivers in a healthcare facility.
FIG. 2 schematically illustrates the caregiver communications system of FIG. 1 that includes a mobile application server and at least one mobile device.
FIG. 3 schematically illustrates an example of a mobile caregiver application installed on the mobile device of FIG. 2.
FIG. 4 illustrates an example of a user interface displayed on the mobile device of FIG. 2, the user interface including a recent interactions tab.
FIG. 5 illustrates another example of a user interface displayed on the mobile device of FIG. 2, the user interface including an interactions timeline.
FIG. 6 illustrates an example of a method of managing caregiver communications that can be performed by the mobile application server of FIG. 2.
FIG. 7 illustrates another example of a method of managing caregiver communications that can be performed by the mobile device of FIG. 2.
FIG. 8 illustrates an example of an invitation generated by the mobile application server of FIG. 2 and received by the mobile devices of FIG. 2.
FIG. 9 illustrates an example of a method of managing caregiver communications that can be performed by the mobile device or the mobile application server of FIG. 2.
FIG. 10 illustrates another example of a user interface displayed on the mobile device of FIG. 2, the user interface displaying a directory that includes recent interactions.
FIG. 11 illustrates an example of a user interface displayed on the mobile device of FIG. 2, the user interface allowing a caregiver to filter a directory by recent interactions.

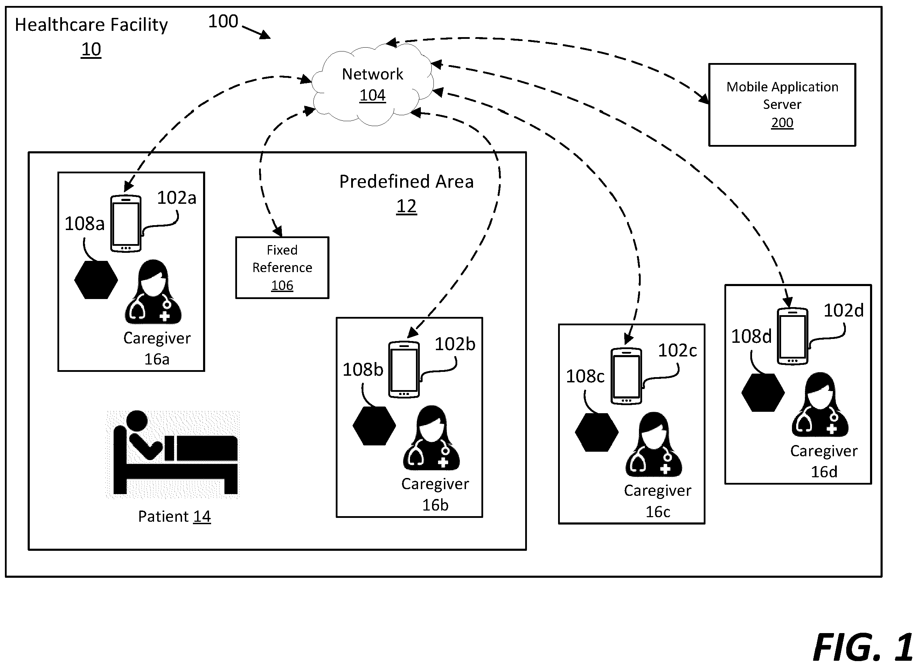

FIG. 1 illustrates an example of a caregiver communications system 100 for managing communications between caregivers 16 in a healthcare facility 10. As an illustrative example, the healthcare facility 10 can be a hospital, nursing home, long term care facility, or similar type of facility. As another illustrative example, the caregivers 16 can include nurses, physician assistants, doctors, medical specialists, and the like.

As shown in FIG. 1, caregivers 16a and 16b are located within a predefined area 12 within the healthcare facility 10, while caregivers 16c and 16d are located outside the predefined area 12. In the example shown in FIG. 1, a patient 14 is also located in the predefined area 12.

In the example shown in FIG. 1, the healthcare facility 10 is shown as having a single predefined area for illustrative purposes. However, the healthcare facility 10 can include more than one predefined area such as a plurality of predefined areas.

In certain examples, the predefined area 12 is defined based on a predetermined proximity to a fixed reference point 106. In the example shown in FIG. 1, caregivers 16a and 16b are within a predetermined proximity of the fixed reference point 106, while caregivers 16c and 16d are outside of the predetermined proximity of the fixed reference point 106. In certain examples, the fixed reference point is a sensor, which will be described in more detail below.

In certain examples, the predetermined proximity to the fixed reference point 106 demarcates the predefined area 12 within the healthcare facility 10 such that the predefined area 12 is a patient room, a pre-operative or post-operative holding area, an operating room, a waiting room, or other type of area within the healthcare facility 10. As another illustrative example, the predetermined proximity to the fixed reference point 106 demarcates a unit within the healthcare facility 10 such as a med/surg unit, an intensive care unit (ICU), a neonatal intensive care unit (NICU), pediatric intensive care unit (PICU), medical intensive care unit (MICU), and the like.

As an illustrative example, the predetermined proximity to the fixed reference point 106 demarcates a patient room such that the caregivers 16a and 16b are within the same patient room. As a further illustrative example, the caregiver 16a can be a nurse who is providing care to the patient 14 inside the patient room such as by obtaining vital signs, administering medications, or helping the patient use the bathroom, and the caregiver 16b can be a medical specialist such as a cardiologist who is making a scheduled visit to see the patient 14 inside the patient room.

In certain example, the predefined area 12 can have one or more physical boundaries such as walls that physically separate it from other areas of the healthcare facility 10. In other examples, the predefined area 12 does not include a physical boundary such that the predefined area 12 is not physically separated from other areas of the healthcare facility 10.

In examples where the fixed reference point 106 is a sensor, the predefined area 12 can include a single sensor, or can include more than one sensor such as a plurality of sensors. In such examples, the predefined area 12 can be defined based on a predetermined proximity to a plurality of sensors. Alternatively, in some examples, the predefined area 12 can be defined based on a fixed reference point that is equidistant to a plurality of sensors.

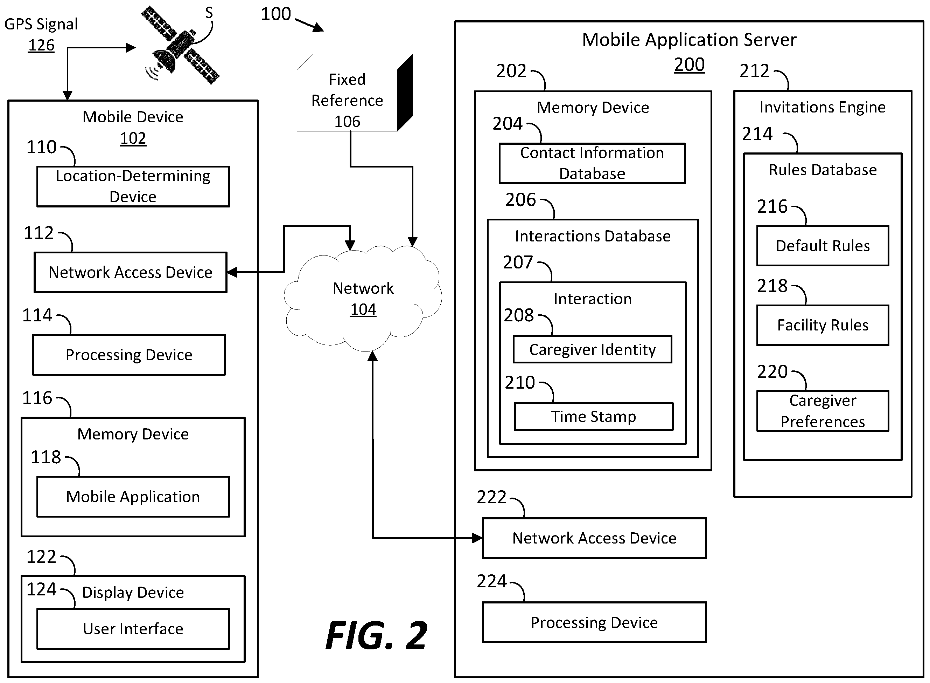

As shown in FIG. 1, each caregiver 16 carries a mobile device 102 that operates a mobile caregiver application 118 (shown in FIG. 2) for managing communications between caregivers 16 over a communications network 104. The mobile caregiver application 118 allows caregivers to use their mobile devices 102 to conduct voice, video, and text messaging between themselves, monitor alerts and calls from assigned patients, and perform additional tasks during their shift in the healthcare facility 10. In some examples, the mobile devices 102 are smartphones, tablet computers, or other types of portable computing devices.

The mobile devices 102 are connected to the communications network 104. In the example shown in FIG. 1, the communications network 104 is located inside the healthcare facility 10 such that the communications network 104 is a local network. Alternatively, the communications network 104 can be an external network such as a cellular network.

The communications network 104 can include any type of wired or wireless connections or any combinations thereof. Examples of wireless connections include broadband cellular network connections such as 4G or 5G. In some examples, wireless connections inside the healthcare facility 10 are accomplished using Wi-Fi, ultra-wideband (UWB), Bluetooth, radio frequency identification (RFID), and similar types of wireless connections.

As shown in FIG. 1, a mobile application server 200 is also connected to the communications network 104. The mobile application server 200 facilitates communications between the caregivers 16, and will be described in more detail with reference to FIG. 2.

As further shown in FIG. 1, each caregiver 16 also carries a badge 108. In certain examples, the badges 108 are used to automatically identify and track the location of the caregivers 16 inside the healthcare facility 10. For example, the badges 108 can include wireless real-time locating systems (RTLS) tags that are detectable by a sensor. In examples where the fixed reference point 106 is a sensor, the fixed reference point 106 can detect and receive wireless signals from RTLS tags to determine the location of the caregivers 16. As an illustrative example, the fixed reference point 106 can detect radio frequency (RF) signals, optical (e.g., infrared) signals, or acoustic (e.g., ultrasound) signals from the badges 108.

In examples where the fixed reference point 106 is a sensor, the fixed reference point 106 can be attached to a wall or ceiling of the predefined area 12, or to an item associated with the patient 14 in the predefined area 12, such as a hospital bed or patient monitor.

In certain examples, the fixed reference point 106 operates to receive short range ID signals from the badges 108 that indicate the presence of the caregivers 16 inside the predefined area 12. The ID signals from the badges 108 are then relayed by the fixed reference point 106 to the mobile application server 200 via the communications network 104.

As another example, an ID signal emitted from the fixed reference point 106 can be detected by the mobile devices 102, and then relayed by the mobile devices 102 to the mobile application server 200 via the communications network 104. Alternatively, an ID signal emitted from the fixed reference point 106 can be received by the badges 108, and then relayed by the badges 108 back to the mobile application server 200 via the communications network 104.

In further examples, a plurality of fixed reference point 106 are included throughout the healthcare facility 10, and ID signals from the badges 108 are received by the plurality of fixed reference point 106. The locations of the caregivers 16 can then be estimated using one or more locating algorithms, such as trilateration, multilateration, or triangulation.

In alternative examples, the fixed reference points 106 are video cameras. In such examples, the mobile application server 200 receives video streams and can use facial recognition technology to identify the caregivers 16 and their location, and to determine whether one or more caregivers are located in the predefined area 12 at the same time.

FIG. 2 schematically illustrates the caregiver communications system 100. In FIG. 2, a mobile device 102, a fixed reference point 106, the mobile application server 200, and the communications network 104 are shown. FIG. 2 further shows a satellite S that provides GPS signals 126 to the mobile device 102. As described above, the mobile device 102 operates a mobile caregiver application 118 for managing voice, video, and text messaging communications over the communications network 104 between the caregivers 16 in the healthcare facility 10.

In at least some examples, the mobile device 102 includes a location-determining device 110, a network access device 112, a processing device 114, a memory device 116, and a display device 122. Other examples may include additional, different, or fewer components. For example, in some instances the mobile device 102 may also include a recording device such as a microphone or camera that operates to record audio or video content.

The location-determining device 110 is a device that determines the location of the mobile device 102. In some examples, the location-determining device 110 uses one or more of the following technologies: Global Positioning System (GPS) technology which may receive the GPS signals 126 from the satellites S, cellular triangulation technology, network-based location identification technology, Wi-Fi positioning systems technology, and combinations thereof.

The network access device 112 operates to communicate with other computing devices over one or more networks, such as the communications network 104. Examples of the network access device 112 include wired network interfaces and wireless network interfaces.

The processing device 114 is an example of a processing unit such as a central processing unit (CPU). The processing device 114 can include one or more central processing units (CPU). In some examples, the processing device 114 can include one or more digital signal processors, field-programmable gate arrays, or other electronic circuits.

The memory device 116 operates to store data and instructions for execution by the processing device 114, including the mobile caregiver application 118. The memory device 116 includes at least some form of computer-readable media. Computer readable media include any media that can be accessed by the mobile device 102. By way of example, computer-readable media include computer readable storage media and computer readable communication media.

Computer readable storage media includes volatile and nonvolatile, removable and non-removable media implemented in any device configured to store information such as computer readable instructions, data structures, program modules, or other data. Computer readable storage media can include, but is not limited to, random access memory, read only memory, electrically erasable programmable read only memory, flash memory, and other memory technology, including any medium that can be used to store information that can be accessed by the mobile device 102. The computer readable storage media is non-transitory.

Computer readable communication media embodies computer readable instructions, data structures, program modules or other data in a modulated data signal such as a carrier wave or other transport mechanism and includes any information delivery media. The term "modulated data signal" refers to a signal that has one or more of its characteristics set or changed in such a manner as to encode information in the signal. By way of example, computer readable communication media includes wired media such as a wired network or direct-wired connection, and wireless media such as acoustic, radio frequency, infrared, and other wireless media. Combinations of any of the above are within the scope of computer readable media.

In some examples, the display device 122 is a touchscreen that operates to receive inputs from a caregiver. In such examples, the display device 122 operates as both a display device and a user input device. In some examples, the display device 122 displays a user interface 124 generated by the mobile caregiver application 118, which is stored in the memory device 116. In some examples, the display device 122 is not a touch screen.

The mobile caregiver application 118 is configured to communicate with the mobile application server 200 over the communications network 104 to receive interactions with other caregivers and staff members determined from the location and movement of the mobile device 102 relative to the location and movement of other mobile devices in the healthcare facility 10. In some examples, the mobile caregiver application 118 receives from the mobile application server 200 invitations to store contact information of the other caregivers and staff members based on the interactions. For example, a first caregiver who is a nurse can receive an invitation to store the contact information of a second caregiver who is a medical specialist temporarily visiting the healthcare facility 10, and who is responsible for providing care to a common patient.

The mobile application server 200 operates to determine the interactions from the location and movement of the mobile device 102 relative to the location and movement of other mobile devices in the healthcare facility 10, and to provide the interactions to the mobile device 102 for display. The mobile application server 200 also operates to provide the invitations to the mobile device 102 for storing the contact information of other caregivers and staff members based on the interactions determined from the location and movement of the mobile device 102 relative to the location and movement of other mobile devices in the healthcare facility 10.

The mobile application server 200 includes a memory device 202, a network access device 222, and a processing device 224. The memory device 202, network access device 222, and processing device 224 may be similar to the processing device 114, memory device 116, and network access device 112, respectively, which are described above.

As shown in FIG. 2, the memory device 202 includes a contact information database 204. In certain examples, the contact information database 204 stores the contact information of the caregivers 16 such as phone number, email address, office/desk location, and the like. The contact information database 204 also stores contact information of additional staff members in the healthcare facility 10 such as administrators, staff members, and the like.

The memory device 202 further includes an interactions database 206 that stores the interactions 207 between two or more caregivers in the healthcare facility 10. An "interaction" is defined herein as an event when two or more caregivers 16 are located in the same area within the healthcare facility 10. For example, FIG. 1 shows an interaction between the caregivers 16a and 16b in the predefined area 12. An interaction can include more than two caregivers such as when three, four, or more caregivers are all simultaneously located in the same predefined area. As shown in FIG. 2, the mobile application server 200 can record a caregiver identity 208 and a time stamp 210 for each interaction 207 stored in the interactions database 206. In some examples, each interaction 207 may also include a location of where the interaction took place.

In certain examples, an interaction 207 can include events when two or more caregivers 16 visit the same predefined area within a predetermined time window, but are not located in the same predefined area at the same time. For example, an interaction can include when a first caregiver enters a predefined area (e.g., a patient room) and then leaves, and within a predetermined time window (e.g., five minutes), a second caregiver enters the same predefined area such that the first and second caregivers were never simultaneously located in the same predefined area, but their visits were within the predetermined time window.

In some examples, the interactions 207 are determined by the mobile application server 200 receiving data from the fixed reference points 106 located in the predefined areas. In examples where the fixed reference points 106 are sensors, an interaction 207 can be determined from data received from the fixed reference points 106 that indicates two or more caregivers 16 are located within a predefined area of the healthcare facility 10.

In examples where the fixed reference points 106 are sensors that detect and receive wireless signals from the RTLS tags on the badges 108 worn by the caregivers 16, the fixed reference points 106 can transmit the detected wireless signals to the mobile application server 200 for analysis. For example, the mobile application server 200 can use the wireless signals from the RTLS tags to determine the location of the caregivers 16 in the healthcare facility, and thereby determine an interaction 207 when two or more caregivers are located in the same area.

In alternative examples where the fixed reference points 106 are video cameras, the fixed reference points 106 can transmit captured video streams over the communications network 104 to the mobile application server 200 for analysis. For example, the mobile application server 200 can use facial recognition to identify the caregivers 16 and their location, and thereby determine an interaction 207 when two or more caregivers are located in the same area.

In further examples, the mobile application server 200 determines the interactions 207 from data received over the communications network 104 from mobile devices 102. For example, an interaction 207 can be determined from GPS signals 126 received from the mobile devices 102 that indicate two more caregivers 16 as being located within a predefined area of the healthcare facility 10. As another example, ID signals emitted from the fixed reference points 106 can be received by the mobile devices 102, and then transmitted by the mobile devices 102 to the mobile application server 200 via the communications network 104.

In further examples, the mobile device 102 of a first caregiver can detect the presence of a mobile device 102 of a second caregiver, and vice versa, using one or more wireless technologies such as Wi-Fi, ultra-wideband (UWB), Bluetooth, radio frequency identification (RFID), or similar technologies. The mobile devices 102 transmit data to the mobile application server 200 for identifying an interaction 207 between two or more caregivers.

As another example, the mobile application server 200 determines the interactions 207 from data received from the badges 108. For example, ID signals emitted from the fixed reference points 106 can be received by the badges 108, and then relayed by the badges 108 to the mobile application server 200 via the communications network 104.

The mobile application server 200 identifies and stores the interactions 207 between caregivers in the healthcare facility 10. The interactions 207 can include interactions between caregivers who have never before interacted in the healthcare facility 10, or interactions between caregivers who infrequently or irregularly interact with one another in the healthcare facility 10. For example, the movements of the caregivers 16 can be monitored over time to determine movement patterns, and thereby determine whether certain caregivers infrequently or irregularly interact with one another. A predetermined period of time for determining infrequent or irregular interactions can be set for two weeks, one month, three months, six months, and the like.

The mobile application server 200 further includes an invitations engine 212 that sends to the mobile device 102 invitations to store the contact information of another caregiver based on at least an interaction 207 with the caregiver in the healthcare facility 10. For example, the invitations engine 212 can send an invitation to the mobile device 102 of a first caregiver, when an interaction 207 with a second caregiver indicates that the first caregiver has never before interacted with the second caregiver, or has infrequently or irregularly interacted with the second caregiver. In some examples, in addition to being based on the interactions 207, the invitations may further be based on satisfaction of one or more rules from a rules database 214.

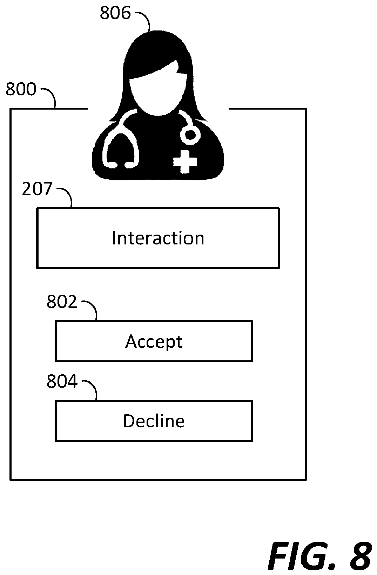

FIG. 8 illustrates an example of an invitation 800 generated by the invitations engine 212 and received by the mobile devices 102 of a first caregiver. The invitation 800 includes an interaction 207, which may include the caregiver identity 208 of a second caregiver, the time stamp 210 of when the interaction 207 took place, and any patients for which both caregivers share responsibility. In some examples, the interaction 207 may also identify a location of where the interaction took place. Also, the invitation 800 can include a profile image 806 of the second caregiver. This can help the first caregiver remember the interaction, such as with who, when, and where the interaction took place.

As further shown in FIG. 8, the invitation 800 includes an icon 802 which the first caregiver can select to accept the invitation 800, and an icon 804 which the first caregiver can select to decline the invitation 800. In some examples, the invitation 800 does not include the icon 804 such that the first caregiver can simply ignore the invitation without having to affirmatively decline the invitation. The invitation 800 can be displayed as a notification on a lock screen of the mobile device 102, which the caregiver can tap to open the mobile caregiver application 118 on the mobile device 102 to either or accept or decline the invitation.

In some examples, the invitation 800 includes the contact information of the second caregiver acquired from the contact information database 204. When the first caregiver accepts the invitation 800 by selecting the icon 802, the second caregiver is added as a contact of the first caregiver, and the mobile caregiver application 118 installed on the mobile device 102 of the first caregiver stores or otherwise provides access to the contact information of the second caregiver.

In alternative examples, the invitation 800 does not include the contact information of the second caregiver. In such examples, when the first caregiver accepts the invitation 800 by selecting the icon 802, another invitation is sent to the mobile device 102 of the second caregiver requesting the second caregiver to share their contact information with the first caregiver. When the second caregiver accepts the invitation, the mobile application server 200 pulls the contact information of the second caregiver from the contact information database 204, and sends the contact information to the mobile device 102 of the first caregiver. When the second caregiver declines the invitation, the mobile application server 200 does not share the contact information of the second caregiver with the mobile device 102 of the first caregiver.

In the example shown in FIG. 2, the rules database 214 can include default rules 216, facility rules 218, and caregiver preferences 220 that define one or more rules that must be satisfied in order to send the invitation 800 to the mobile device 102 of the first caregiver. The default rules 216 are general rules that govern communications between the caregivers 16. The facility rules 218 are rules set by the healthcare facility 10 and may supplement the default rules such as by adding additional rules, or overriding one or more of the default rules 216.

The rules defined by the default rules 216 or facility rules 218 can be based on the interaction 207 between the first and second caregivers in the healthcare facility 10. As an illustrative example, an invitation 800 to store the contact information of the second caregiver is sent to the mobile device 102 of the first caregiver only when the first caregiver interacts with the second caregiver within the predefined area 12 of the healthcare facility 10, such as a patient room. In such an example, the first caregiver does not receive an invitation 800 to store the contact information of the second caregiver when the first caregiver interacts with the second caregiver outside of the predefined area 12, such as in a cafeteria of the healthcare facility 10. Advantageously, this can also avoid sending invitations when there is a meaningless interaction, such as when the first caregiver interacts with the second caregiver in a common area, and there is no need for the first caregiver to store the contact information of the second caregiver.

As another example, an invitation 800 to store the contact information of the second caregiver is sent to the mobile device 102 of the first caregiver only after the first caregiver has interacted with the second caregiver beyond a predetermined number of times (e.g., only after the third interaction) in the healthcare facility 10. In such an example, the first caregiver does not receive an invitation 800 to store the contact information of the second caregiver when the first caregiver interacts with the second caregiver for the first time in the healthcare facility 10. In some examples, a predetermined number of interactions must occur within a predetermined time frame (e.g., three interactions with the second caregiver within one week). Advantageously, this can avoid sending invitations when there is a one-off interaction, such as when the first caregiver interacts in the healthcare facility 10 with the second caregiver only once, and never again.

The rules defined by the default rules 216 or facility rules 218 can also block a first caregiver from receiving the contact information of a second caregiver based on at least one of assigned department or unit within the healthcare facility 10, assigned patients, patient rooms, or patient areas within the healthcare facility 10, or scheduled shifts in the healthcare facility 10.

As an illustrative example, the one or more rules defined by the default rules 216 or facility rules 218 can allow sharing contact information only between caregivers who are in the same department or unit within the healthcare facility. As another illustrative example, the rules can allow sharing contact information only between caregivers who are assigned to at least one common patient or who are assigned to at least one common patient room or patient area within the healthcare facility 10. As another illustrative example, the rules can allow sharing contact information only between caregivers who have overlapping shifts in the healthcare facility 10.

In some examples, the electronic medical record (EMR) of a patient can be checked to determine whether the first and second caregivers are caring for the same patient, or whether the second caregiver would be likely to interact with the first caregiver based on one or more medical conditions of the patient identified from the patient's EMR. In such examples, the rules can help anticipate or predict a need for the first caregiver to contact the second caregiver.

The caregiver preferences 220 include rules based on the preferences of the caregivers. For example, the caregiver preferences 220 can include whether a caregiver is interested or not in receiving the invitations 800 to add other caregivers as contacts based on their interactions in the healthcare facility 10, or whether the caregiver allows or does not allow sharing their contact information with the other caregivers in the healthcare facility 10.

As another example, the default rules 216 or facility rules 218 can prevent the second caregiver from declining an invitation from the first caregiver. In this example, the first caregiver can add the second caregiver as a contact without requiring approval from the second caregiver.

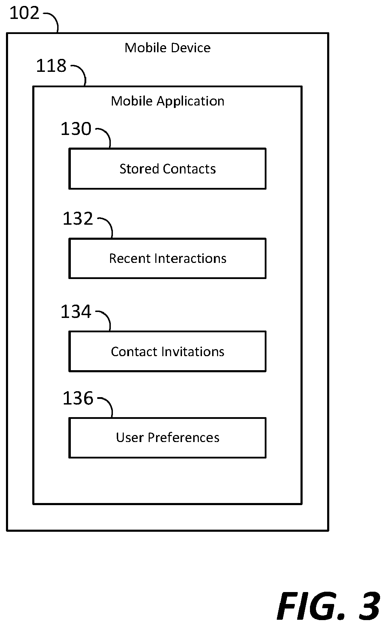

FIG. 3 schematically illustrates an example of the mobile caregiver application 118 installed on a mobile device 102 of a caregiver. The mobile caregiver application 118 includes stored contacts 130, recent interactions 132, contact invitations 134, and user preferences 136. Other examples may include additional, different, or fewer components.

The stored contacts 130 include contact information of caregivers and staff members in the healthcare facility 10 who are known by the caregiver. The contact information can include a mobile phone number, office phone number, email address, and the like. In some examples, the stored contacts 130 are stored on the memory device 116 of the mobile device 102. In other examples, the stored contacts 130 are stored in the memory device 202 of the mobile application server 200, and are accessible by the mobile device 102. In further examples, the stored contacts 130 are stored both in the memory device 116 of the mobile device 102 and in the memory device 202 of the mobile application server 200, such as for backup cloud storage.

The recent interactions 132 include interactions 207 with other caregivers and staff members in the healthcare facility 10 that are pulled from the interactions database 206 stored in the mobile application server 200. The recent interactions 132 can be updated in real-time as the caregiver carries the mobile device 102 around the healthcare facility 10 during their shift.

In some examples, the interactions 207 are filtered such that the recent interactions 132 includes only interactions with other caregivers and staff members who are not in the stored contacts 130. In other examples, the recent interactions 132 can include interactions with caregivers and staff members who are in the stored contacts 130, and also those who are not.

The contact invitations 134 include the invitations 800 sent from the mobile application server 200 to the mobile device 102, based on the recent interactions 132 and/or satisfaction of one or more rules such as the rules included in the rules database 214. As described above, the invitations 800 can be displayed as notifications on the lock screen of the mobile device 102, which the caregiver can tap to open the mobile caregiver application 118 on the mobile device 102 to either or accept or decline the invitations. Alternatively, the invitations 800 can be accessed by the caregiver through the contact invitations 134.

The user preferences 136 store personalized information of the caregiver. For example, the user preferences 136 can include whether the caregiver is interested or not in receiving contact invitations to connect with other caregivers based on their interactions in the healthcare facility 10, or whether the caregiver allows or does not allow the sharing of their contact information with other caregivers in the healthcare facility 10.

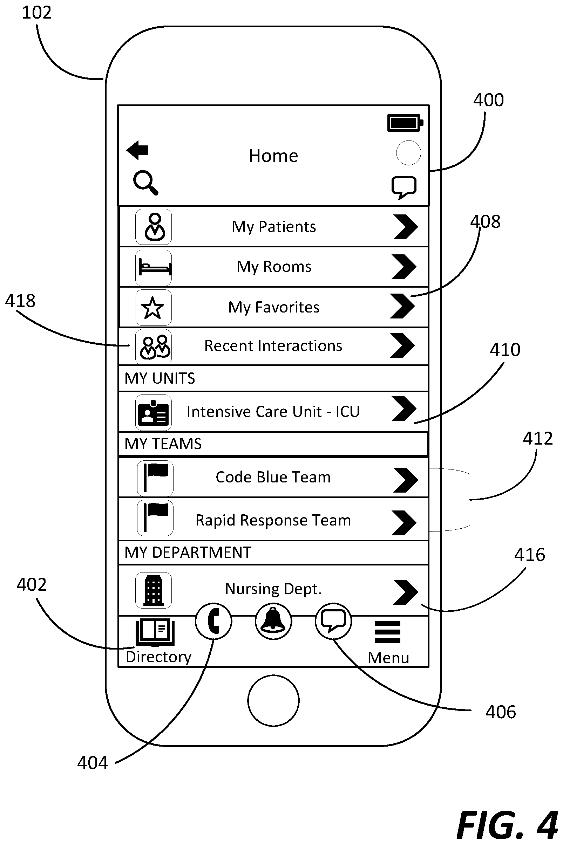

FIG. 4 illustrates an example of a user interface 400 generated by the mobile caregiver application 118 for display on the mobile device 102. Referring now to FIGS. 3 and 4, the caregiver can access the stored contacts 130 by selecting the directory icon 402 on the user interface 400. Also, the caregiver can select a phone call icon 404 or a text message icon 406 to call or text message, respectively, another caregiver or staff members in the stored contacts 130.

Additionally, the stored contacts 130 can be organized into different categories for quick reference. For example, the stored contacts 130 are organized into my favorites 408, one or more units 410 to which the caregiver belongs (e.g., Intensive Care Unit (ICU)), one or more teams 412 to which the caregiver belongs (e.g., Code Blue Team, Rapid Response Team), and/or the department 416 to which the caregiver belongs (e.g., Nursing Department).

As shown in FIG. 4, the user interface 400 includes a recent interactions tab 418 that when selected by the caregiver, causes the mobile caregiver application 118 to display the recent interactions 132. In some examples, the recent interactions 132 are displayed as a list that is provided in chronological order with the most recent interactions being listed first.

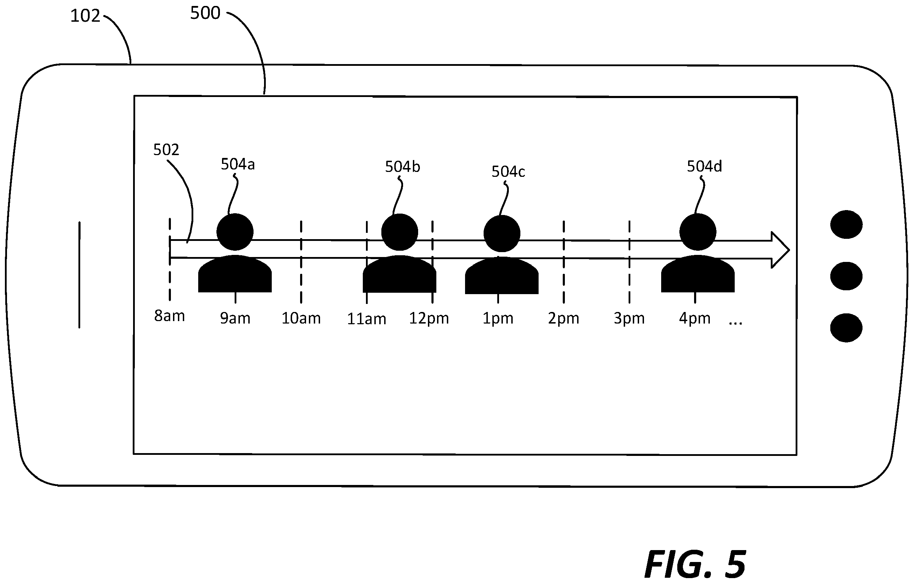

FIG. 5 illustrates an example of a user interface 500 generated by the mobile caregiver application 118 for display on a mobile device 102 associated with a caregiver. The user interface 500 can be generated when the caregiver selects the recent interactions tab 418 shown in FIG. 4. In this example, the user interface 500 includes an interaction timeline 502 for displaying in chronological order the interactions the caregiver had with other caregivers and staff members in the healthcare facility 10. The interaction timeline 502 can start from when the caregiver started their shift (e.g., 8 am), and can terminate when the caregiver ends their shift for a particular day. In some examples, the mobile caregiver application 118 can generate and display an interaction timeline 502 for each day the caregiver worked for a given week or month.

The interaction timeline 502 include icons 504 that represent the interactions 207 with other caregivers and staff members in the healthcare facility 10. The icons 504 are displayed based on the caregiver identity 208 and time stamp 210 of each interaction 207 (see FIG. 2). In the example shown in FIG. 5, a first icon 504a is displayed around the 9am hour mark to indicate that there was an interaction with another caregiver around this time. Similarly, a second icon 504b is displayed between the 11am and 12pm hour marks, a third icon 504c is displayed at the 1pm hour mark, and a fourth icon 504d is displayed at the 4pm hour mark.

In some examples, the interactions 207 are filtered such that the icons 504 are displayed only for caregivers and staff members identified from the recent interactions 132 who are not in the stored contacts 130. In such examples, icons 504 represent interactions with caregivers and staff members who may not be known by the caregiver associated with the mobile device 102 because the caregiver has never before interacted or infrequently interacts with them.

In other examples, the icons 504 are displayed for caregivers and staff members identified from the recent interactions 132 regardless of whether or not they are in the stored contacts 130. In such examples, the icons 504 may be displayed differently to distinguish between caregivers and staff members who are in the stored contacts 130 from those who are not. For example, the icons 504 for caregivers and staff members who are in the stored contacts 130 can be displayed as clear profile images, and/or can include the names of the caregivers and staff members, as well as additional identification details obtained from the stored contacts 130.

The icons 504 for caregivers and staff members who are not in the stored contacts 130 can be displayed as stock images, or as blurred profile images to indicate that they may not be known by the caregiver associated with the mobile device 102. In some examples, the icons 504 for caregivers and staff members who are not in the stored contacts 130 can include generic labels such as their job position or function, and/or assigned department or unit within the healthcare facility 10, but not specific identification data such as their full name and contact information. In some further examples, the icons 504 for caregivers and staff members who are not in the stored contacts 130 can include their first name, and first letter of their last name.

One or more of the icons 504 displayed along the interaction timeline 502 can be selected to receive the contact information of the caregiver or person associated with the icon. In some examples, the contact information is automatically obtained from the contact information database 204 and added to the stored contacts 130 upon selection of an icon.

In other examples, an invitation is sent to the caregiver or person associated with the selected icon, and the caregiver or person can accept or decline the invitation to share their contact information with the caregiver associated with the mobile device 102. When the caregiver or person associated with the selected icon accepts the invitation, the mobile application server 200 pulls the contact information of the caregiver or person from the contact information database 204, and sends the contact information to the mobile device 102. When the caregiver or person associated with the selected icon declines the invitation, the mobile application server 200 does not send the contact information to the mobile device 102.

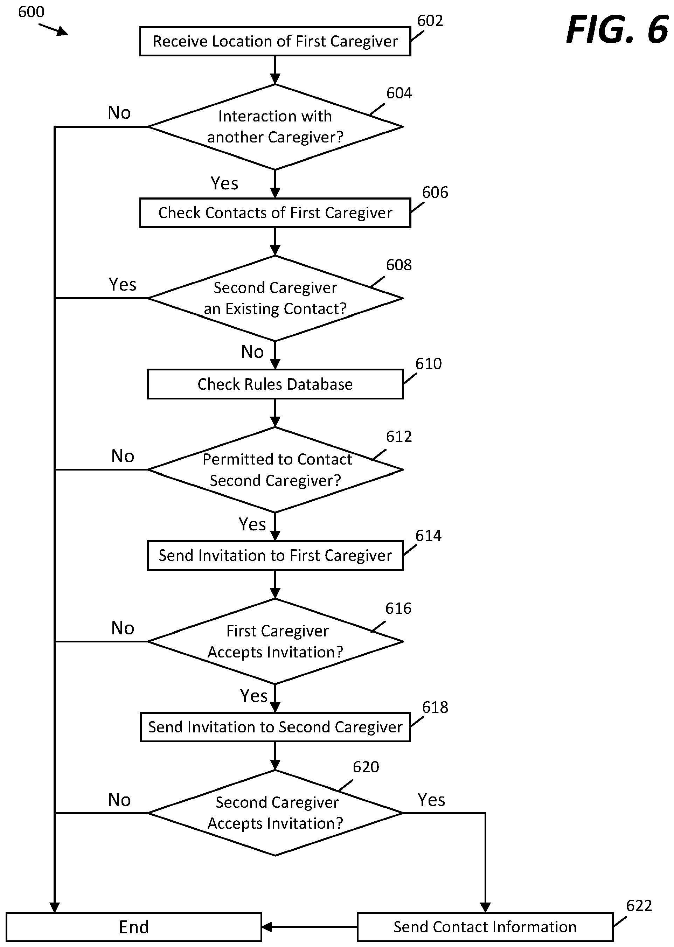

FIG. 6 illustrates an example of a method 600 of managing caregiver communications. The method 600 can be performed by the mobile application server 200. The method 600 includes an operation 602 of receiving a location of a mobile device 102 associated with a first caregiver. The location of the mobile device 102 associated with the first caregiver can be obtained in accordance with any of the examples described above. In some examples, the location of the mobile device 102 associated with the first caregiver is determined to be inside a predefined area based on a predetermined proximity to a fixed reference point.

Next, the method 600 includes an operation 604 of determining whether an interaction 207 with another caregiver is detected. As described above, an interaction 207 is detected when a second caregiver is located in the same predefined area as the first caregiver or has visited the same predefined area as the first caregiver within a predetermined time window. An interaction 207 is detected by comparing the location of the second caregiver with the location of the first caregiver to determine whether the first and second caregivers are simultaneously located in the same predefined area at the same time. The location of the second caregiver can be obtained in accordance with any of the examples described above.

Also, an interaction 207 is detected when the second caregiver visits the same predefined area as the first caregiver within the predetermined time window by comparing the time stamp 210 from when the first caregiver visited the predefined area with the time stamp 210 from when the second caregiver visited the predefined area. When the difference between the time stamps 210 is less than the predetermined time window, an interaction 207 is detected.

When an interaction with another caregiver is not detected (i.e., "No" at operation 604), the method 600 ends. When an interaction with another caregiver is detected (i.e., "Yes" at operation 604), the method 600 proceeds to operation 606.

Operation 606 includes checking the stored contacts 130 of the first caregiver to determine whether the contact information of a second caregiver in the interaction detected in operation 604 is already stored in the stored contacts 130 of the first caregiver. If the second caregiver is already known by the first caregiver, an invitation to store the contact information of the second caregiver is not needed. As an illustrative example, operation 606 can include using the mobile phone number of the second caregiver to determine whether there is a matching mobile phone number in the stored contacts 130 of the first caregiver.

When the second caregiver is an existing contact of the first caregiver (i.e., "Yes" at operation 608), the method 600 ends. When the second caregiver is not an existing contact of the first caregiver (i.e., "No" at operation 608), the method 600 proceeds to operation 610.

Operation 610 includes checking the rules database 214 to determine whether the first caregiver is permitted to receive the contact information of the second caregiver. As described above, the rules database 214 includes default rules 216 and facility rules 218 that define rules that may allow or block the first caregiver from adding the second caregiver to the stored contacts 130 of the first caregiver. The one or more rules can be based on at least one of assigned department or unit within the healthcare facility 10, assigned patients, patient rooms, or patient areas within the healthcare facility 10, or scheduled shifts in the healthcare facility 10.

Also, the rules database 214 includes caregiver preferences 220, which include rules based on the preferences of the caregivers. For example, the caregiver preferences 220 can block the first caregiver from receiving the contact information of the second caregiver when the second caregiver does not allow sharing their contact information with the other caregivers in the healthcare facility 10, based on the user preferences 136 of the second caregiver.

When the first caregiver is not permitted to receive the contact information of the second caregiver (i.e., "No" at operation 612), the method 600 ends. When the first caregiver is permitted to receive the contact information of the second caregiver (i.e., "Yes" at operation 612), the method 600 proceeds to operation 614, which is to send an invitation to the mobile device 102 of the first caregiver to add the second caregiver as a contact.

As described above, the first caregiver can accept or decline the invitation. When the first caregiver declines the invitation (i.e., "No" at operation 616), the method 600 ends. In some examples, when the first caregiver accepts the invitation (i.e., "Yes" at operation 616), the first caregiver is allowed to add the second caregiver as a new contact in the stored contacts 130, and the mobile caregiver application 118 installed on the mobile device 102 of the first caregiver stores or otherwise provides access to the contact information of the second caregiver.

In alternative examples, operation 614 can include automatically adding the second caregiver as a contact of the first caregiver without sending an invitation to the first caregiver to accept or decline. Thus, when the first caregiver accesses their stored contacts 130 (see FIG. 3) such as by selecting the directory icon 402 on the user interface 400 (see FIG. 4), a list of contacts will include the second caregiver without requiring any input from the first caregiver.

In some alternative examples, when the first caregiver accepts the invitation (i.e., "Yes" at operation 616), the method 600 includes an operation 618 of sending a second invitation to the mobile device 102 of the second caregiver requesting the second caregiver to share their contact information with the first caregiver. When the second caregiver accepts the second invitation (i.e., "Yes" at operation 620), the method 600 includes an operation 622 of sending the contact information to the mobile device 102 of the first caregiver. When the second caregiver declines the second invitation (i.e., "No" at operation 620), the method 600 ends.

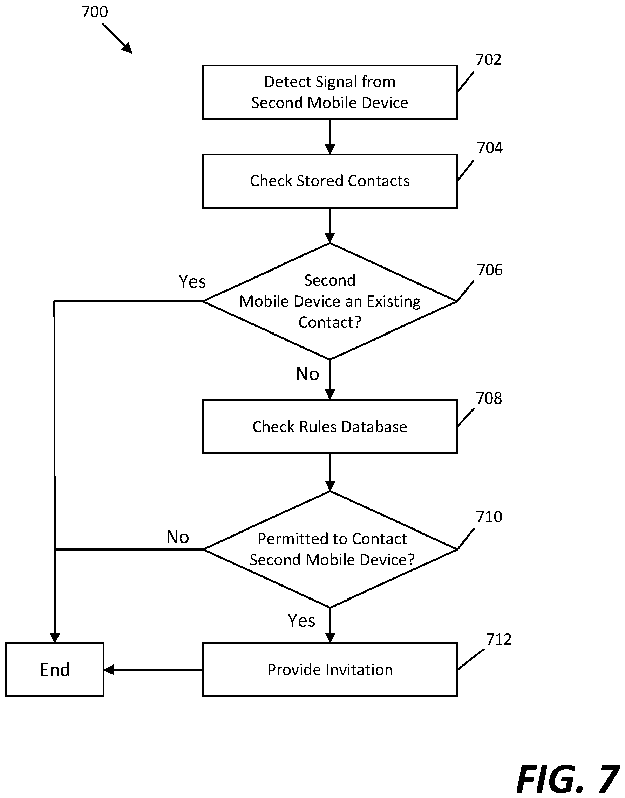

FIG. 7 illustrates another example of a method 700 of managing caregiver communications. The method 700 can be performed by a mobile device 102 of a first caregiver without using the fixed reference point 106 shown in FIGS. 1 and 2.

The method 700 includes an operation 702 of detecting a signal from a mobile device 102 of a second caregiver. Operation 702 is performed using one or more wireless technologies described above such as Wi-Fi, ultra-wideband (UWB), Bluetooth, radio frequency identification (RFID), or similar technologies which can detect the presence of a mobile device 102 of a second caregiver when in close proximity of the mobile device of the first caregiver. The signal detected from the mobile device 102 of the second caregiver includes data that identifies the mobile device 102 of the second caregiver such as a mobile phone number or a device name.

Next, the method 700 proceeds to operation 704 of determining whether the second caregiver is in the stored contacts 130 the first caregiver. For example, the mobile phone number or device name of the mobile device 102 of the second caregiver is used to determine whether there is a matching phone number or device name in the stored contacts 130 the first caregiver.

When the second caregiver is determined to be an existing contact in the stored contacts 130 of the first caregiver (i.e., "Yes" at operation 706), the method 700 ends. When the second caregiver is determined not to be an existing contact in the stored contacts 130 of the first caregiver (i.e., "No" at operation 706), the method 700 proceeds to operation 708.

Operation 708 includes checking the rules database 214 to determine whether the first caregiver is permitted to receive the contact information of the second caregiver. In some examples, the mobile device 102 of the first caregiver communicates with the mobile application server 200 over the communications network 104 to access the rules database 214. Alternatively, the rules database 214 can be stored locally on the memory device 116 of the mobile device 102.

As described above, the rules database 214 includes default rules 216 and facility rules 218 that allow or block the first caregiver from adding the second caregiver to the stored contacts 130 of the first caregiver. The one or more rules can be based on at least one of assigned department or unit within the healthcare facility 10, assigned patients, patient rooms, or patient areas within the healthcare facility 10, or scheduled shifts in the healthcare facility 10.

Also, the rules database 214 includes caregiver preferences 220, which include rules based on the preferences of the caregivers. For example, the caregiver preferences 220 can block the first caregiver from receiving the contact information of the second caregiver when the second caregiver does not allow sharing their contact information with the other caregivers in the healthcare facility 10, based on the user preferences 136 of the second caregiver. As another illustrative example, the default rules 216 or facility rules 218 can prevent the second caregiver from declining an invitation from the first caregiver, and thereby allow the first caregiver to add the second caregiver as a contact without requiring approval from the second caregiver.

When the first caregiver is not permitted to receive the contact information of the second caregiver (i.e., "No" at operation 710), the method 700 ends. When the first caregiver is permitted to receive the contact information of the second caregiver (i.e., "Yes" at operation 710), the method 700 proceeds to operation 712, and provides an invitation to connect with the second caregiver. As described above, the first caregiver can accept or decline the invitation. When the invitation is accepted, the mobile device 102 of the first caregiver allows the first caregiver to store the contact information of the second caregiver in the stored contacts 130.

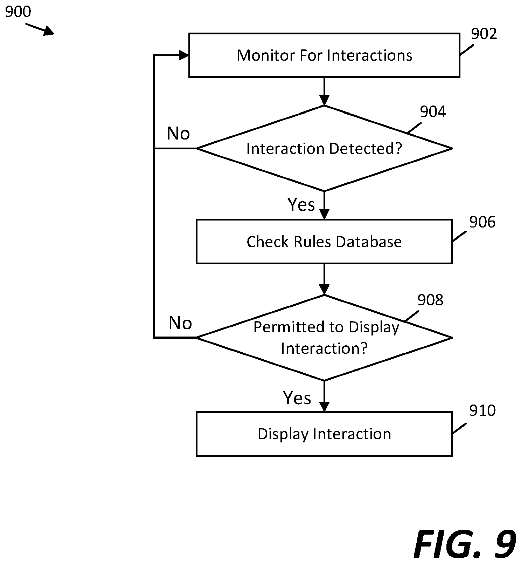

FIG. 9 illustrates an example of a method 900 of managing caregiver communications. The method 900 can be performed by the mobile device 102. Alternatively, the method 900 can be performed by the mobile application server 200.

The method 900 includes an operation 902 of monitoring for interactions between caregivers and staff members. Operation 902 can be performed by the mobile device 102 or the mobile application server 200 in accordance with the examples described above.

Next, the method 900 includes an operation 904 of determining whether an interaction between two or more caregivers or staff members is detected. When an interaction is not detected (i.e., "No" at operation 904), the method 900 returns to operation 902 and continues monitoring for interactions. When an interaction is detected (i.e., "Yes" at operation 904), the method 900 proceeds to operation 906, which includes checking the rules database 214.

Operation 906 includes checking the rules database 214 to determine whether display of the interaction is permitted on a mobile device associated with a first caregiver. In some examples, the mobile device of the first caregiver communicates with the mobile application server 200 over the communications network 104 to access the rules database 214. Alternatively, the rules database 214 can be stored locally on the memory device 116 of the mobile device. As described above, the rules database 214 includes default rules 216 and facility rules 218.

Next, the method 900 includes an operation 908 of determining whether a display of the interaction is permitted. Determining whether the display of the interaction is permitted can be based on one or more shared characteristics between the two or more caregivers or staff members identified in the interaction such as assigned department or unit within the healthcare facility 10, assigned patients, patient rooms, or patient areas within the healthcare facility 10, or scheduled shifts in the healthcare facility 10.

When the display of the interaction is not permitted (i.e., "No" at operation 908), the method 900 returns to operation 902 and continues monitoring for interactions. When display of the interaction is permitted (i.e., "Yes" at operation 908), the method 900 proceeds to operation 910, which includes displaying the interaction on the mobile device of the first caregiver when the first caregiver has the one or more shared characteristics.

In accordance with operation 910, the first caregiver may want to contact a second caregiver they recently met (e.g., a traveling nurse), but not add the second caregiver to their stored contacts 130. Thus, operation 910 does not include storing the second caregiver as a new contact. Instead, the first caregiver can view the interaction with the second caregiver in a menu or directory displayed on their mobile device, and can contact the second caregiver without storing the second caregiver as a new contact. In some examples, operation 910 includes displaying the interaction in a menu or directory of interactions that is sorted in chronological order, allowing the caregiver to view the most recent interactions first.

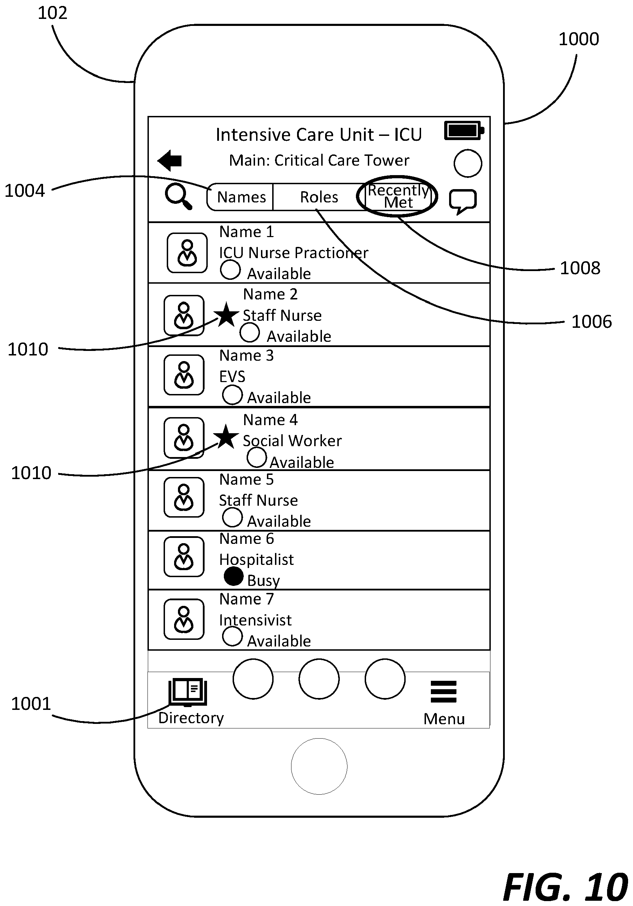

FIG. 10 illustrates an example of a user interface 1000 that can be displayed on the mobile device 102 in accordance with the operations of the method 900. The user interface 1000 can be displayed in response to a caregiver selecting the directory tab 1001. The user interface 1000 includes a directory of members 1002 assigned to a certain unit or department within the healthcare facility 10 (e.g., "Intensive Care Unit - ICU"). The user interface 1000 allows a caregiver to select an icon 1004 to view the directory of members 1002 in alphabetical order by name, and to select an icon 1006 to view or filter the directory of members 1002 by role (e.g., "ICU Nurse Practitioner", "Social Worker", "Intensivist", and the like).

Certain members in the directory of members 1002 are identified by an icon 1010 as having had a recent interaction with the caregiver associated with the mobile device 102. The caregiver can then select these members from the directory of members 1002 to contact them without having to store the members as new contacts in their stored contacts 130.

Additionally, the user interface 1000 allows a caregiver to select an icon 1008 to view the directory of members 1002 based on recent interactions. When the caregiver selects the icon 1008, a list of members identified as having had a recent interaction with the caregiver associated with the mobile device 102 are displayed on the mobile device 102 in chronological order.

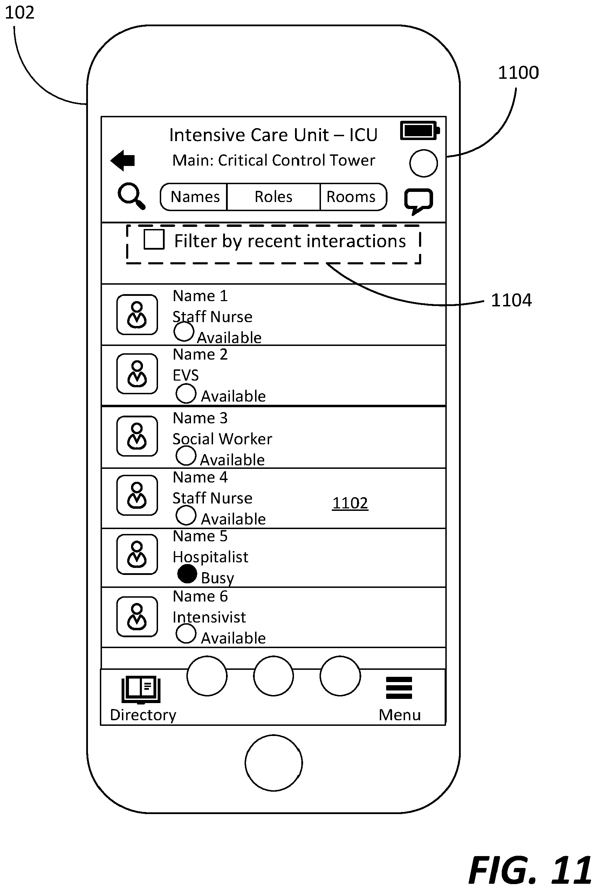

FIG. 11 illustrates another example of a user interface 1100 displayed on the mobile device 102. The user interface 1100 is similar to the user interface 1000 described above, in that the user interface 1100 includes a directory of members 1102 assigned to a certain unit or department within the healthcare facility 10 (e.g., "Intensive Care Unit - ICU"). Additionally, the user interface 1100 provides an option 1104 that allows the caregiver to filter the directory of members 1102 by recent interactions. Thereafter, the caregiver can select a member from directory of members 1102, who is identified as having a recent interaction with the caregiver, to contact the member without having to store them as a new contact in their stored contacts 130.

The various embodiments described above are provided by way of illustration only and should not be construed to be limiting in any way. Various modifications can be made to the embodiments described above without departing from the true spirit and scope of the disclosure.

Aspects of the present disclosure are defined in the following numbered clauses:
1. A system for managing caregiver communications in a healthcare facility, the system comprising: at least one processing device; and a memory device storing instructions which, when executed by the at least one processing device, cause the system to: detect an interaction between a first caregiver and a second caregiver; determine whether the second caregiver is a contact of the first caregiver; determine whether the first caregiver is permitted to receive the contact of the second caregiver based on at least the interaction in the healthcare facility; and send an invitation to a mobile device of the first caregiver, the invitation allowing the first caregiver to accept or decline adding the contact of the second caregiver when the first caregiver is permitted to receive the contact of the second caregiver.
2. The system of clause 1, wherein determine whether the first caregiver is permitted to receive the contact information of the second caregiver is based on at least one of a unit, a team, a department, a patient, a patient room, and a shift assigned to the first and second caregivers.
3. The system of clause 1 or 2, wherein determine whether the first caregiver is permitted to receive the contact information of the second caregiver is based on the second caregiver's preferences.
4. The system of clause 1, 2, or 3, wherein the memory device stores further instructions which, when executed by the at least one processing device, cause the system to: display an interaction timeline on the mobile device of the first caregiver, the interaction timeline including interactions with other caregivers in the healthcare facility.
5. The system of clause 4, wherein the interaction timeline is filtered to include only interactions with the other caregivers who are not contacts of the first caregiver.
6. The system of clause 4, wherein the interactions with the other caregivers who are contacts of the first caregiver are displayed on the interaction timeline differently from the interactions with the other caregivers who are not contacts of the first caregiver.
7. The system of any preceding clause, wherein the interaction is detected by receiving signals from one or more fixed reference points indicating the first and second caregivers are located together in a predefined area, or have visited the predefined area within a predetermined time window.
8. The system of clause 7, wherein the one or more fixed reference points obtain the signals from badges worn by the first and second caregivers.
9. The system of any preceding clause, wherein the interaction is detected by receiving a video stream and using facial recognition to identify the first and second caregivers as being located together in a predefined area, or having visited the predefined area within a predetermined time window.
10. The system of any preceding clause, wherein the interaction is detected by receiving data from the mobile device of the first caregiver and a mobile device of the second caregiver.
11. The system of clause 10, wherein the data includes GPS signals indicating the first and second caregivers are located together in a predefined area, or have visited the predefined area within a predetermined time window.
12. The system of clause 10, wherein the data includes signals obtained from fixed reference points indicating the first and second caregivers are located together in a predefined area, or have visited the predefined area within a predetermined time window.
13. The system of any preceding clause, wherein the interaction is detected by receiving data from the mobile device of the first caregiver detecting a mobile device of the second caregiver using at least one of Wi-Fi, ultra-wideband (UWB), Bluetooth, and radio frequency identification (RFID).
14. The system of any preceding clause, wherein the interaction is detected by receiving data from badges worn by the first and second caregivers, and the data includes signals obtained from fixed reference points indicating the first and second caregivers are located together in a predefined area, or have visited the predefined area within a predetermined time window.
15. A mobile device for managing caregiver communications in a healthcare facility, the mobile device comprising: at least one processing device; and a memory device storing instructions which, when executed by the at least one processing device, cause the mobile device to: detect a signal from a second mobile device associated with a caregiver; determine whether the caregiver is a contact; determine whether the mobile device is permitted to receive the contact of the caregiver based on at least an interaction with the caregiver in the healthcare facility; and provide an invitation to add the contact of the caregiver when the mobile device is permitted to receive the contact of the caregiver.
16. The mobile device of clause 15, wherein the signal from the mobile device associated with the caregiver is detected using at least one of Wi-Fi, ultra-wideband (UWB), Bluetooth, and radio frequency identification (RFID).
17. The mobile device of clause 15 or 16, wherein the memory device stores further instructions which, when executed by the at least one processing device, cause the mobile device to: display an interaction timeline including interactions with other caregivers in the healthcare facility, the interactions on the interaction timeline displayed in chronological order.
18. The mobile device of clause 17, wherein the interaction timeline is filtered to include only interactions with the other caregivers who are not existing contacts.
19. The mobile device of clause 17, wherein the interactions with the other caregivers who are existing contacts are displayed on the interaction timeline differently from the interactions with the other caregivers who are not existing contacts.
20. A method for managing caregiver communications in a healthcare facility, the method comprising: detecting an interaction between a first caregiver and a second caregiver; determining whether the second caregiver is a contact of the first caregiver; determining whether sharing the contact of the second caregiver is permitted based on at least one of a unit, a team, a department, a patient, a patient room, and a shift assigned to the first and second caregivers; and sending an invitation to a mobile device of the first caregiver, the invitation allowing the first caregiver to add the contact of the second caregiver.

## Claims

1. A system for managing caregiver communications in a healthcare facility, the system comprising:
at least one processing device; and
a memory device storing instructions which, when executed by the at least one processing device, cause the system to:
detect an interaction between a first caregiver and a second caregiver;
determine whether the second caregiver is a contact of the first caregiver;
determine whether the first caregiver is permitted to receive the contact of the second caregiver based on at least the interaction in the healthcare facility; and
send an invitation to a mobile device of the first caregiver, the invitation allowing the first caregiver to accept or decline adding the contact of the second caregiver when the first caregiver is permitted to receive the contact of the second caregiver.

2. The system of claim 1, wherein determine whether the first caregiver is permitted to receive the contact information of the second caregiver is based on:
at least one of a unit, a team, a department, a patient, a patient room, and a shift assigned to the first and second caregivers; and/or
the second caregiver's preferences.

3. The system of claim 1 or 2, wherein the memory device stores further instructions which, when executed by the at least one processing device, cause the system to:
display an interaction timeline on the mobile device of the first caregiver, the interaction timeline including interactions with other caregivers in the healthcare facility.

4. The system of claim 3, wherein the interaction timeline is filtered to include only interactions with the other caregivers who are not contacts of the first caregiver, or wherein the interactions with the other caregivers who are contacts of the first caregiver are displayed on the interaction timeline differently from the interactions with the other caregivers who are not contacts of the first caregiver.

5. The system of claim 3 or 4, wherein interactions displayed on the interaction timeline are selectable to receive the contact information of the caregiver or person associated with the respective interaction.

6. The system of any preceding claim, wherein the interaction is detected by receiving signals from one or more fixed reference points indicating the first and second caregivers are located together in a predefined area, or have visited the predefined area within a predetermined time window,
optionally wherein the or each fixed reference point comprises a sensor and/or wherein the one or more fixed reference points are configured to obtain the signals from badges worn by the first and second caregivers, and
further optionally wherein the badges comprise a wireless real-time locating systems RTLS tag configured to be detectable by a, or the, sensor.

7. The system of any preceding claim, wherein the interaction is detected by receiving a video stream and using facial recognition to identify the first and second caregivers as being located together in a predefined area, or having visited the predefined area within a predetermined time window.

8. The system of any preceding claim, wherein the interaction is detected by receiving data from the mobile device of the first caregiver and a mobile device of the second caregiver.

9. The system of claim 8, wherein the data includes at least one of:
GPS signals indicating the first and second caregivers are located together in a predefined area, or have visited the predefined area within a predetermined time window; and
signals obtained from fixed reference points indicating the first and second caregivers are located together in a predefined area, or have visited the predefined area within a predetermined time window.

10. The system of any preceding claim, wherein the interaction is detected by receiving data from the mobile device of the first caregiver detecting a mobile device of the second caregiver using at least one of Wi-Fi, ultra-wideband (UWB), Bluetooth, and radio frequency identification (RFID), and/or wherein the interaction is detected by receiving data from badges worn by the first and second caregivers, and the data includes signals obtained from fixed reference points indicating the first and second caregivers are located together in a predefined area, or have visited the predefined area within a predetermined time window.

11. The system of any preceding claim, wherein the invitation includes a, or the, interaction,
optionally wherein the interaction includes at least one of a caregiver identity of the second caregiver, a time stamp of when the interaction took place, any patients for which both caregivers share responsibility, a location of where the interaction took place, and a profile image of the second caregiver.

12. The system of any preceding claim, wherein the memory device stores further instructions which, when executed by the at least one processing device, cause the system to:
check an electronic medical record (EMR) of a patient to determine whether the first and second caregivers are caring for the same patient, or whether the second caregiver would be likely to interact with the first caregiver based on one or more medical conditions of the patient identified from the patient's EMR

13. The system of any preceding claim, wherein determine whether the first caregiver is permitted to receive the contact of the second caregiver based on at least the interaction in the healthcare facility comprises determining whether one or more rules is satisfied,
optionally wherein the one or more rules comprises default rules, facility rules, and caregiver preferences,
further optionally wherein the default rules or the facility rules comprises at least one of:
the invitation to store the contact information of the second caregiver is sent to the mobile device of the first caregiver only when the first caregiver interacts with the second caregiver within the predefined area of the healthcare facility, such as a patient room;
the invitation is sent only after the first caregiver has interacted with the second caregiver beyond a predetermined number of times in the healthcare facility,
preferably, the predetermined number of interactions must occur within a predetermined time frame; and
blocking a first caregiver from receiving the contact information of a second caregiver based on at least one of assigned department or unit within the healthcare facility, assigned patients, patient rooms, or patient areas within the healthcare facility, or scheduled shifts in the healthcare facility,
preferably sharing contact information is allowed only between caregivers who are in the same department or unit within the healthcare facility, and/or who are assigned to at least one common patient, and/or who are assigned to at least one common patient room or patient area within the healthcare facility, and/or between caregivers who have overlapping shifts in the healthcare facility.

14. A mobile device for managing caregiver communications in a healthcare facility, the mobile device comprising:
at least one processing device; and
a memory device storing instructions which, when executed by the at least one processing device, cause the mobile device to:
detect a signal from a second mobile device associated with a caregiver;
determine whether the caregiver is a contact;
determine whether the mobile device is permitted to receive the contact of the caregiver based on at least an interaction with the caregiver in the healthcare facility; and
provide an invitation to add the contact of the caregiver when the mobile device is permitted to receive the contact of the caregiver,
optionally, wherein the signal from the mobile device associated with the caregiver is detected using at least one of Wi-Fi, ultra-wideband (UWB), Bluetooth, and radio frequency identification (RFID), and/or
wherein the memory device stores further instructions which, when executed by the at least one processing device, cause the mobile device to:
display an interaction timeline including interactions with other caregivers in the healthcare facility, the interactions on the interaction timeline displayed in chronological order, and
optionally wherein the interaction timeline is filtered to include only interactions with the other caregivers who are not existing contacts, or wherein the interactions with the other caregivers who are existing contacts are displayed on the interaction timeline differently from the interactions with the other caregivers who are not existing contacts.

15. A method for managing caregiver communications in a healthcare facility, the method comprising:
detecting an interaction between a first caregiver and a second caregiver;
determining whether the second caregiver is a contact of the first caregiver;
determining whether sharing the contact of the second caregiver is permitted based on at least one of a unit, a team, a department, a patient, a patient room, and a shift assigned to the first and second caregivers; and
sending an invitation to a mobile device of the first caregiver, the invitation allowing the first caregiver to add the contact of the second caregiver.
